(19) Europäisches Patentamt
European Patent Office
Office européen des brevets

(11) Veröffentlichungsnummer: **0 381 010**
**A2**

(12) # EUROPÄISCHE PATENTANMELDUNG

(21) Anmeldenummer: **90101266.6**

(51) Int. Cl.5: **C07C 209/10, C07C 211/52**

(22) Anmeldetag: **23.01.90**

(30) Priorität: **26.01.89 DE 3902239**

(43) Veröffentlichungstag der Anmeldung: **08.08.90 Patentblatt 90/32**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Papenfuhs, Theodor, Dr.**
**Heinrich-Bleicher-Strasse 40**
**D-6000 Frankfurt am Main 50(DE)**
Erfinder: **Hess, Reiner, Dr.**
**Lärchenstrsasse 4**
**D-6200 Wiesbaden(DE)**
Erfinder: **Vorwerk, Edgar, Dr.**
**Breslauer Strasse 41**
**D-6233 Kelkheim (Taunus)(DE)**

(54) **Verfahren zur Herstellung von 2-Nitro-4-trifluormethyl-anilin.**

**Verfahren zur Herstellung von 2-Nitro-4-trifluormethylanilin, dadurch gekennzeichnet, daß man auf 4-Chlor-3-nitro-benzotrifluorid überschüssiges wäßriges Ammoniak bei Temperaturen von etwa 80 bis etwa 150°C, gegebenenfalls in Gegenwart eines Kupferkatalysators, einwirken läßt.**

EP 0 381 010 A2

## Verfahren zur Herstellung von 2-Nitro-4-trifluormethyl-anilin

(57) Die vorliegende Erfindung betrifft ein vorteilhaftes Verfahren zur Herstellung von 2-Nitro-4-trifluormethyl-anilin aus 4-Chlor3-nitro-benzotrifluorid und wäßrigem Ammoniak.

2-Nitro-4-trifluormethyl-anilin stellt ein wertvolles Ausgangsprodukt zur Herstellung von Heilmitteln (DE-OS 24 59 453, FR 2 558 464), Farben und Pflanzenschutzmitteln dar.

Gemäß FR 2 558 464 wird 2-Nitro-4-trifluormethyl-anilin auf folgende Weise hergestellt:

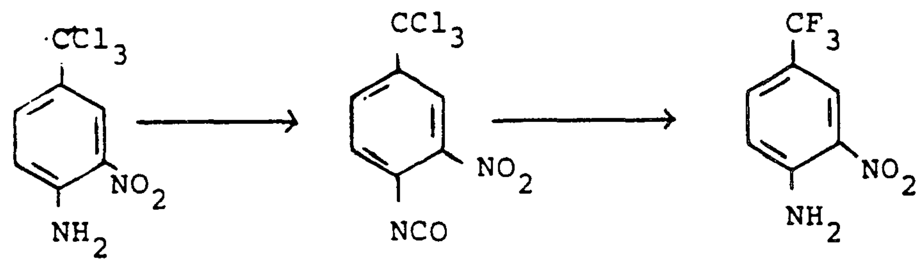

Hierbei muß, um zum Ausgangsprodukt der Synthese, dem 4-Trichloromethyl-2-nitro-phenylisocyanat, zu gelangen, zunächst 4-Trichloromethyl-2-nitro-anilin mit Phosgen zum Isocyanat umgesetzt werden. Einerseits ist diese Umsetzung wegen der Giftigkeit des Phosgens technisch schwer zu handhaben, andererseits ist das Ausgangsprodukt 4-Trichloromethyl-2-nitro-anilin nur auf aufwendigem Wege und technisch unbefriedigend herzustellen und wegen seiner Neigung zur Selbstkondensation nicht lagerstabil.

In der Folgestufe wird 4-Trichloromethyl-2-nitro-phenyl isocyanat in flüssigem Fluorwasserstoff zu 2-Nitro-4-trifluormethyl-anilin umgesetzt. Dieses Vorgehen erfordert einen großen technischen Aufwand, beispielsweise das Arbeiten in korrosionsunempfindlichen Druckbehältern. Ferner muß nach jedem Reaktionszyklus zur Aufarbeitung der Fluorwasserstoff vollständig destillativ entfernt werden, um das Reaktionsprodukt zu isolieren.

Ein anderer Reaktionsweg zur Herstellung von 2-Nitro-4-trifluormethyl-anilin wird in J. Org. Chem., Vol. 42, Nr. 3 (1977) und in der DE-OS 24 59 453 beschrieben, wobei wie folgt vorgegangen wird:

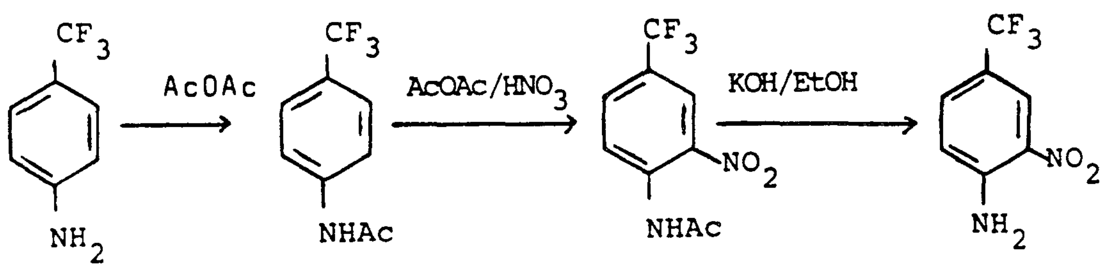


4-Trifluormethyl-anilin, dessen Herstellung und Handhabung in mehreren Veröffentlichungen (FR 2 558 464) als sehr schwierig beschrieben wird, wird in Acetanhydrid acyliert, mit Salpetersäure in Essigsäureanhydrid nitriert und anschließend in ethanolischer Kalilauge deacyliert.

Im Rahmen der zweiten Reaktionsstufe muß zur Isolierung des noch acylierten Nitroproduktes die Essigsäure vollständig neutralisiert oder destillativ entfernt werden. Da sich die DE-OS 24 59 453 auf die Herstellung der 1,2-Diamino-Verbindung bezieht, geht man auf die aufwendige Isolierung auf dieser Stufe nicht ein, sondern verweist auf die Verwendung des Rohproduktes für die Folgestufe. Da weder Ausbeute- noch Reinheitsangaben gemacht werden, und das 2-Nitro-4-trifluormethyl-anilin nur als Öl erhalten wird, dessen Identität nur durch IR-Spektroskopie bestätigt wird, kann man von mäßigen Ausbeuten bei schlechter Reinheit ausgehen, die zudem noch durch eine technisch undurchführbare Etherextraktion erhalten werden.

Hinzukommt, daß die Nitrierung in Essigsäure/Acetanhydrid durch die Entstehung des zersetzlichen Acetylnitrats (HOUBEN-WEYL, Methoden der Organischen Chemie, Bd. X/1, Seiten 484 und 757) einen sicherheitstechnisch nur schwer beherrschbaren Reaktionsweg darstellt, dessen Durchführung mit einem hohen Aufwand an apparativen Sicherheitseinrichtungen verbunden ist. Im einschlägigen Patentbeispiel der DE-OS 24 59 453 wird zudem bei 25° C gearbeitet, obwohl sich Acetylnitrat schon oberhalb 18° C merklich

zersetzt.

Insgesamt ergibt sich durch die relativ große Zahl von Reaktionsschritten, den dadurch resultierenden Chemikalien-und Energieaufwand und die aufwendigen Sicherheitsinvestitionen in der Nitrierungsstufe ein technisch nur schwer realisierbares Verfahren.

Es bestand somit ein Bedarf für ein vorteilhaftes Verfahren zur Herstellung von 2-Nitro-4-trifluormethyl-anilin in sehr guten Ausbeuten und hoher Reinheit, das nicht mit den Nachteilen der zum Stand der Technik zählenden Verfahren behaftet ist.

Es wurde nun überraschenderweise gefunden, daß man 2-Nitro-4-trifluormethylanilin in sehr guten Ausbeuten und hoher Reinheit herstellen kann, indem man auf 4-Chlor-3-nitro-benzotrifluorid überschüssiges wäßriges Ammoniak bei Temperaturen von etwa 80 bis etwa 150 °C, vorzugsweise von etwa 100 bis etwa 120 °C, gegebenenfalls in Gegenwart eines Kupferkatalysators, einwirken läßt.

Was den anzuwendenden Überschuß von wäßrigem Ammoniak anbelangt, so ist es zweckmäßig, etwa 200 bis etwa 1000 Mol-%, vorzugsweise etwa 400 bis etwa 700 Mol-% Ammoniak, bezogen auf das eingesetzte 4-Chlor-3-nitro-benzotrifluorid, einwirken zu lassen. Die Konzentration der eingesetzten wäßrigen Ammoniaklösung bewegt sich zwischen etwa 20 und etwa 30, vorzugsweise zwischen etwa 22 und etwa 28 Gewichtsprozent Ammoniak pro Liter Lösung.

An Kupferkatalysatoren sind beispielsweise Kupfer(II)-carbonat oder Kupfer(I)-oxid geeignet.

Das erfindungsgemäße Verfahren kann sowohl diskontinuierlich als auch kontinuierlich durchgeführt werden. Es versteht sich von selbst, daß man je nach angewandter Ammoniakkonzentration und Temperatur unter Druck arbeiten muß.

Beim erfindungsgemäßen Verfahren kann vorteilhafterweise die nach der Umsetzung anfallende ammoniakalische Mutterlauge durch Verdünnen mit Wasser, welches zur Produktwäsche dient, anteilmäßig, vorzugsweise zu zwei Dritteln, in den Folgeansatz zurückgeführt werden, wobei lediglich Ammoniak und Ausgangsverbindung, in der jeweils erforderlichen bzw. gewünschten Menge zudosiert werden müssen, um einen neuen Reaktionszyklus beginnen zu können. Auf diese Weise wird erreicht, daß die Konzentration des sich bildenden Ammoniumchlorids in der Mutterlauge nie zu groß wird, was nämlich zur Folge hätte, daß die Mutterlauge wegen Ausfallen des Salzes komplett ausgeschleust werden müßte.

Vorteilhaft für das erfindungsgemäße Verfahren ist auch, daß man vom technisch Verfügbaren 4-Chlor-benzotrifluorid durch ein technisch ausgereiftes und sicherheitstechnisch problemloses Verfahren (Nitrierung mit einem Schwefelsäure/Salpetersäure-Gemisch in Schwefelsäure (HOUBEN-WEYL, Methoden der Organischen Chemie, Bd. X/1, Seite 482)) zur Ausgangsverbindung (4-Chlor-3-nitro-benzotrifluorid) gelangt.

Es ist als überraschendes Merkmal des erfindungsgemäßen Verfahrens zu erachten, daß das 4-ständige Chloratom der Ausgangsverbindung, das lediglich durch die 2-ständige Nitrogruppe aktiviert ist, mittels überschüssigem wäßrigen Ammoniak durch die Aminogruppe bei relativ milden Bedingungen, so bei bevorzugten Temperaturen von nur etwa 100 bis etwa 120 °C, ausgetauscht werden kann.

Aus der Literatur bekannte, vergleichbare Umsetzungen von Verbindungen mit aktivierten Chloratomen (HOUBEN-WEYL, Methoden der Organischen Chemie, Band XI/1, Seiten 63-64 (1957)) ließen nämlich wesentlich drastischere Reaktionsbedingungen (Temperaturen von 180 - 250 °C, Reaktionszeiten von 20 - 24 Stunden) erwarten.

Demgegenüber gelingt es beim erfindungsgemäßen Verfahren, den Austausch des Chloratoms durch die Aminogruppe bei den genannten relativ niedrigen Temperaturen innerhalb eines Zeitraums von nur 5 - 6 Stunden, ohne Zusatz von Lösemitteln oder Löslichkeitsvermittlern, wie Alkoholen oder Phasentransferkatalysatoren, in sehr guter Ausbeute bei ausgezeichneter Produktreinheit vorzunehmen.

Die nachstehenden Beispiele dienen zur Erläuterung der Erfindung ohne sie darauf zu beschränken.

**Beispiel 1**

In einem 5-l-Stahlautoklav werden 285 g 4-Chlor-3-nitro-benzotrifluorid (2,85 Mol) und 1250 g Ammoniaklösung 22 %ig (16,2 Mol Ammoniak) vorgelegt. Der Autoklav wird verschlossen und unter Rühren werden bei Raumtemperatur weitere 114 g Ammoniak (6,7 Mol) aus einem Druckbehälter zugesetzt.

Anschließend wird das Reaktionsgemisch auf 115 °C erhitzt und 6 Stunden bei dieser Temperatur gehalten. Hierbei stellt sich ein Druck von 11 bar ein. Nach Ablauf dieser Zeitspanne rührt man, bis sich Raumtemperatur eingestellt hat, und öffnet dann das nun drucklose Reaktionsgefäß.

Die ausgetragene Suspension des kristallinen Produktes in der Mutterlauge wird abgesaugt, zweimal mit jeweils 300 ml Wasser gewaschen und getrocknet. Hierbei werden insgesamt etwa 1800 g Mutter- und Waschlauge erhalten, die anteilig in den Folgeansatz rückgeführt werden können.

Man erhält 581 g 2-Nitro-4-trifluormethyl-anilin, entsprechend einer Ausbeute von 99 % der Theorie, mit

einem Schmelzpunkt von 106 - 107 °C und einer Reinheit von >98 % (HPLC).

**Beispiel 2**

In einem 5-l-Edelstahlautoklav werden 644 g (2,85 Mol) 4-Chlor-3-nitro-benzotrifluorid und 1250 g Mutterlauge aus dem Voransatz (11,8 Mol Ammoniak) vorgelegt.

Nach Verschließen der Apparatur werden weitere 190 g (11,2 Mol) Ammoniak aus einem Druckbehälter zugegeben. Das weitere Vorgehen entspricht Beispiel 1.

Man erhält 575 g 2-Nitro-4-trifluormethyl-anilin, entsprechend einer Ausbeute von 98 % der Theorie, mit einem Schmelzpunkt von 106 - 107,5 °C und einer Reinheit von >98 % (HPLC).

**Ansprüche**

1. Verfahren zur Herstellung von 2-Nitro-4-trifluormethyl-anilin, dadurch gekennzeichnet, daß man auf 4-Chlor-3-nitro-benzotrifluorid überschüssiges wäßriges Ammoniak bei Temperaturen von etwa 80 bis etwa 150 °C, gegebenenfalls in Gegenwart eines Kupferkatalysators, einwirken läßt.

2. Verfahren nach Anspruch 1, dadurch gekennzeichnet, daß man etwa 200 bis etwa 1000 Mol-% Ammoniak auf 1 Mol 4-Chlor-3-nitro-benzotrifluorid einwirken läßt.

3. Verfahren nach Anspruch 1, dadurch gekennzeichnet, daß man etwa 400 bis etwa 700 Mol-% Ammoniak auf 1 Mol 4-Chlor-3-nitro-benzotrifluorid einwirken läßt.

4. Verfahren nach mindestens einem der Ansprüche 1 - 3, dadurch gekennzeichnet, daß man überschüssiges wäßriges Ammoniak bei Temperaturen von etwa 100 bis 120 °C einwirken läßt.

5. Verfahren nach mindestens einem der Ansprüche 1 - 4, dadurch gekennzeichnet, daß man in Gegenwart von Kupfer(II)-carbonat oder Kupfer(I)-oxid als Katalysator einwirken läßt.

6. Verfahren nach mindestens einem der Ansprüche 1 - 5, dadurch gekennzeichnet, daß man die nach der Umsetzung anfallende ammoniakalische Mutterlauge nach Verdünnen mit Wasser und Aufstärken mit Ammoniak und Zusatz von Ausgangsverbindung anteilmäßig in den Folgeansatz rückführt.

7. Verfahren nach mindestens einem der Ansprüche 1 bis 6, dadurch gekennzeichnet, daß man kontinuierlich arbeitet.